# EUROPEAN PATENT APPLICATION

(11) **EP 2 067 764 A1**
(43) Date of publication of application: **10.06.2009**
(21) Application number: 08168378.1
(22) Date of filing: 05.11.2008
(51) Int. Cl.: C07C 51/02, C07C 51/41, C07C 59/64, C07C 211/05

(54) **Process for the preparation of pure acitretin**

(30) Priority: 07.11.2007 IT MI20072128
(71) Applicant: SOLMAG S.p.A., 26837 Mulazzano LO (IT)
(72) Inventor: Pizzocaro, Roberta, 20024, GARBAGNATE MILANESE (MI) (IT); Pellegatta, Cesare, 20024, GARBAGNATE MILANESE (MI) (IT)
(74) Representative: Minoja, Fabrizio

(57) **Abstract**

The invention relates to a process for the preparation of substantially pure Acitretin, which comprises the following steps:
a) salifying crude Acitretin with an amine of formula (II)

R₁R₂R₃N (II)

wherein each of R₁, R₂ and R₃ is, independently, hydrogen, a C₁-C₆ alkyl group, a C₃-C₈ cycloalkyl group, a phenyl, morpholino or pyridino group, in the presence of an alcohol or water-alcohol solvent; and
b) adding an organic acid to the salt obtained in step a).

## Description

### Field of the invention

The present invention relates to a process for the purification of Acitretin of formula (I)

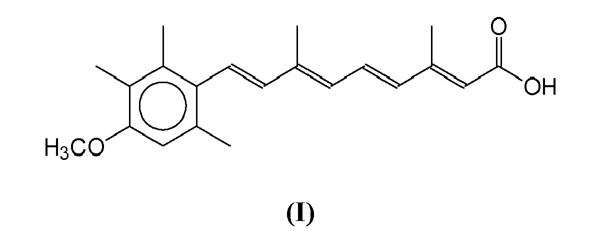

### Technological Background

(2E, 4E, 6E, 8E)-9-(4-Methoxy-2,3,6-trimethylphenyl)-3,7-dimethyl-2,4,6,8- nonatetraenoic acid, commonly known as Acitretin, is a retinoic acid aromatic derivative used in the treatment of severe psoriasis in adults both in monotherapy and combination therapy, or in the after-treatment as a disease stabilizer.

Examples 1-3 of Hoffman-La Roche Patent US 4,105,681 disclose the preparation of Acitretin and provide a mixture of the *cis*- and *trans-*stereoisomers.

Ranbaxy Labs. Ltd. Patent US 7,129,365 discloses a process for the preparation of substantially pure Acitretin, which comprises contacting crude Acitretin with solvents in the presence of a free radicals *scavenger* such as hydroxy anisole butoxide or hydroxy toluene butoxide.

Examples 1 and 2 of the Glenmark Pharm. Ltd. international patent application WO 2007/017720 concern a process for the preparation of substantially pure Acitretin, which involves the formation and recovery of a crude Acitretin sodium or lithium salt and the subsequent hydrolysis of the salt with hydrochloric acid for isolating the desired product.

An alternative method for the preparation substantially pure Acitretin has now been found, which is functionally simple and can easily be scaled-up.

### Disclosure of the invention

It is therefore an object of the present invention to provide a process for the preparation of substantially pure Acitretin, which comprises the following steps:
a) salifying crude Acitretin with an amine of formula (II)

   R₁R₂R₃N (II)

   wherein each of R₁, R₂ and R₃ is, independently, hydrogen, a C₁-C₆ alkyl group, a C₃-C₈ cycloalkyl group, a phenyl, morpholino or pyridino group; in the presence of an alcohol or water-alcohol solvent, and
b) adding an organic acid to the salt obtained in step a).

The reaction mixture of step a) can optionally be added with potassium hydroxide.

According to the present invention, the definition "crude Acitretin" comprises Acitretin in any form; for example, it means Acitretin containing a mixture of various *cis-trans-* isomers as impurities. Crude Acitretin can be prepared, for example, following the teaching of Hoffman-La Roche Patent US 4,105,681.

According to the present invention, the definition "substantially pure Acitretin" means Acitretin comprising less than 2%, preferably less than 1%, most preferably less than 0.5%, of undesired chemical impurities, as determined by HPLC.

According to the present invention, the definition "chemical impurities" means, in particular, *cis-* stereoisomers and Acitretin esters, more particularly (2Z, 4E, 6E, 8E)-9-(4-methoxy-2,3,6-trimethylphenyl)-3,7-dimethyl-2,4,6,8-nonatetraenoic acid and ethyl (2E, 4E, 6E, 8E)-9-(4-methoxy-2,3,6-trimethylphenyl)-3,7-dimethyl-2,4,6,8-nonatetraenoate, as from the monograph in European Pharmacopoeia, 4a Edition, 2002.

According to the present invention, the definition "C₁-C₆ alkyl" comprises, for example, methyl, ethyl, n-propyl, isopropyl, n-butyl, isobutyl, n-pentyl and n-hexyl.

According to the present invention, the definition "C₃-C₈ cycloalkyl" comprises, for example, cyclopropyl, cyclobutyl, cyclopentyl and cyclohexyl.

Preferably, in an amine of formula (II), each R₁, R₂ and R₃ is independently hydrogen or a C₁-C₄ straight alkyl group; more preferably, said amine is selected from triethylamine, diethylamine, dipropylamine and diisopropylamine; most preferably from triethylamine and diethylamine.

The solvent used in step a) is preferably a water-alcohol solvent, in which the alcohol is selected from methanol, ethanol, propanol, isopropanol or mixtures thereof; more preferably from ethanol and methanol.

The organic acid used in step b) is preferably an aliphatic carboxylic acid of formula R₄COOH, wherein R₄ is hydrogen or a C₁-C₄ straight alkyl group, preferably hydrogen or methyl, namely formic or acetic acid.

According to the present invention, step a) is usually carried out adding crude Acitretin to a mixture of an alcohol or water-alcohol solvent and an amine of formula (II) as defined above, stirring until complete dissolution.

According to the present invention, step b) is usually carried out adding the solution from step a) with an organic acid as defined above, until complete precipitation of substantially pure Acitretin, which is then recovered.

According to a preferred aspect, the present invention relates to a process for the preparation of substantially pure Acitretin, which comprises the following steps:
a) salifying crude Acitretin with an amine of formula (II) as defined above wherein each of R₁, R₂ and R₃ is, independently, hydrogen or a C₁-C₆ alkyl group, in the presence of a water-alcohol solvent; and
b) adding an aliphatic carboxylic acid of formula R₄COOH, wherein R₄ is hydrogen or a C₁-C₄ straight alkyl group, to the salt obtained in the above step a).

According to a most preferred aspect, the present invention relates to a process for the preparation of substantially pure Acitretin, which comprises the following steps:
a) salifying crude Acitretin with an amine selected from triethylamine and diethylamine, in the presence of a water-alcohol solvent; and
b) adding an aliphatic carboxylic acid lected from acetic and formic acid to the salt obtained in step a).

The invention is illustrated in greater detail by the following examples.

### EXAMPLES

### Example 1

A mixture of 260 ml of ethanol and 60 ml of water under nitrogen atmosphere is added with 38 g of triethylamine.

50 g of crude Acitretin is poured in the mixture with stirring, keeping stirring at temperature of 15°C-25°C until complete dissolution.

The solution is added with 220 ml of ethanol and slowly with 27 g of acetic acid, with cooling, thereby precipitating Acitretin.

The mixture is left under stirring for not less than 2 hours and the solid is filtered and washed with ethanol, to obtain Pure Acitretin (46 g on average).

### Example 2

A mixture of 400 ml of methanol and 60 ml of water under nitrogen atmosphere is added with 38 g of triethylamine.

50 g of crude Acitretin are poured in the mixture, stirring at a temperature of 15°C-25°C until complete dissolution.

The solution is slowly added with 27 g of acetic acid, with cooling, thereby precipitating Acitretin, which after about 2 hours is separated from reaction mixture by filtration to obtain about 45 g of pure Acitretin.

## Claims

1. A process for the preparation of substantially pure Acitretin, which comprises the following steps:
a) salifying crude Acitretin with an amine of formula (II)
R₁R₂R₃N (II)
wherein each of R₁, R₂ and R₃ is, independently, hydrogen, a C₁-C₁ alkyl group, a C₃-C₈ cycloalkyl group, a phenyl, morpholino or pyridino group, in the presence of an alcohol or water-alcohol solvent; and
b) adding an organic acid to the salt obtained in step a).

2. A process as claimed in claim 1, wherein in the amine of formula (II) each of R₁, R₂ and R₃ is, independently, hydrogen or a C₁-C₆ alkyl group, and the organic acid is an aliphatic carboxylic acid of formula R₄COOH, in which R₄ is hydrogen or a C₁-C₄ straight alkyl group.

3. A process as claimed in claim 2, wherein the amine of formula (II) is diethylamine or triethylamine and the aliphatic carboxylic acid of formula R₄COOH is acetic or formic acid.

4. A process as claimed in any one of claims 1 to 3, wherein the solvent used in step a) is a water-alcohol solvent.

5. A process as claimed in claim 4, wherein the alcohol of the water-alcohol solvent is selected from methanol, ethanol, propanol and isopropanol.

6. A process as claimed in any one of claims 1 to 5, wherein the reaction mixture of step a) is added with potassium hydroxide.
